# EUROPÄISCHE PATENTSCHRIFT

(11) **EP 1 322 277 B1**
(45) Veröffentlichungstag und Bekanntmachung des Hinweises auf die Patenterteilung: **13.02.2008**
(21) Anmeldenummer: 01976261.6
(22) Anmeldetag: 28.09.2001
(51) Int. Cl.: A61K 6/06, C25D 1/14, A61C 5/08, C04B 35/119, C04B 35/622

(54) **DENTALES FORMTEIL UND VERFAHREN ZU DESSEN HERSTELLUNG**
DENTAL MOULDED PART AND METHOD FOR THE PRODUCTION THEREOF
PIECE MOULEE DENTAIRE ET SON PROCEDE DE PRODUCTION

(30) Priorität: 06.10.2000 DE 10049971
(43) Veröffentlichungstag der Anmeldung: 02.07.2003
(73) Patentinhaber: Wieland Dental + Technik GmbH & Co. KG, 75179 Pforzheim (DE)
(72) Erfinder: LAUBERSHEIMER, Jürgen, 76307 Karlsbad (DE)
(74) Vertreter: Patentanwälte Ruff, Wilhelm, Beier, Dauster & Partner
(86) Internationale Anmeldenummer: PCT/EP2001/011220
(87) Internationale Veröffentlichungsnummer: WO 2002/030362

(56) Entgegenhaltungen:
- EP-A1- 0 631 995
- EP-A2- 0 826 642
- WO-A1-98/48084
- WO-A1-99/50480
- US-A- 5 415 748

## Beschreibung

Die Erfindung betrifft ein dentales Formteil, das aus Keramikpartikeln und Brennen des daraus erhaltenen Grünlings herstellbar ist, sowie ein Verfahren zu dessen Herstellung.

Schon immer war Keramik oder "Porzellan" ein attraktiver Werkstoff, um Zähne mit sehr zahnähnlichem Aussehen in Form und Farbe nachzubilden. Seit wissenschaftlich belegt ist, daß Keramik ein chemisch beständiger, korrosionsfester und biokompatibler Werkstoff ist, der zudem noch in schier unendlicher Menge in mineralischer Form verfügbar und mit zahntechnischen Mitteln individueller, paßgenauer Zahnersatz einfach und reproduzierbar herzustellen ist, ist der Durchbruch des Werkstoffes "Dentalkeramik" eingetreten.

Um die einzige Schwäche dieses Werkstoffes, die Bruchempfindlichkeit, zu umgehen, wird zahntechnisch gefertigter Zahnersatz in der Regel schon seit Jahrzehnten als klassischer Werkstoff-Verbund hergestellt, z. B. als sogenannte Metallkeramik. Eine metallkeramische Krone oder Brücke besteht aus einem metallischem Gerüst bzw. Unterbau und einer der Zahnform nachempfundenen sogenannten Verblendung aus Dentalkeramik. Der Unterbau wird beim Einsetzen des Zahnersatzes direkt auf dem nach der zahnärztlichen Präparation verbleibenden Restzahn befestigt und wird oft als (Schutz-)Käppchen bezeichnet. Je nachdem, aus welchem Metall bzw. aus welcher Legierung die Käppchen bestehen und je nach Herstellverfahren (Gießen, Galvanoforming-Verfahren) können Probleme in Form von Korrosion und daraus resultierenden Verfärbungen, Körperunverträglichkeiten und anderes mehr entstehen, weshalb in den letzten Jahren zunehmend Systeme entwickelt werden, um vergleichbare Unterkonstruktionen aus keramischen Materialien herstellen und zahntechnisch weiterverarbeiten zu können.

Es gibt bereits mehrere funktionierende Systeme auf dem Dentalmarkt, bei denen die Keramik-Käppchen beispielsweise durch manuelles Auftragen eines Schlickers auf einen Modellstumpf, anschließendem Sinterbrand sowie nachfolgender Infiltration mit Spezialglas, durch einen Pressvorgang unter Temperatureinwirkung bzw. mehrere Systeme, bei denen die Käppchen aus vorgesintertem Keramikblöcken digital gefräst werden, hergestellt werden. Allen diesen sogenannten Vollkeramik-Systemen ist jedoch gemeinsam, daß die Paßgenauigkeit metallischer Käppchen auf dem Restzahn, ob sie nun gegossen sind oder durch galvanische Prozesse entstehen, in der Regel nicht erreicht werden. Beispielsweise muß beim digitalen Ausfräsen der Käppchen nach einem digital aufgenommenen Datensatz aus festem Material spanabhebend gefräst werden. Das Scannen des Zahnstumpfes und das Fräsen bedingen, je nach der digitalen Auflösung der Systemkomponenten, bereits Ungenauigkeiten. Zudem sind die Systeme in der Anschaffung meist sehr teuer.

Es ist bekannt, dentale Formteile elektrophoretisch abzuscheiden. Die elektrophoretische Formgebung erzeugt aus dispergierten, frei beweglichen Teilen einen Gegenstand mit definierter geometrischer Form unter Ausnutzung der Kraftwirkung eines elektrischen Feldes auf diese Teilchen infolge deren elektrischer Ladung. Dabei ist die Masse des elektrophoretisch abgeschiedenen Materials proportional der angelegten Spannung.

In der WO 99/50480 ist ein Verfahren zur elektrophoretischen Abscheidung von keramischen Partikeln zur Herstellung von Keramikkörpern für dentale Anwendungen beschrieben. Dabei wird zunächst eine Suspension von keramischen Partikeln in einem polaren Lösungsmittel, insbesondere in einem Alkohol, hergestellt. Die Suspension besteht zu mindestens 5 Gew. % aus keramischen Partikeln. Danach wird ein Strom durch die Suspension geleitet, der die Abscheidung der keramischen Partikeln an einer als dentaler Formkörper ausgebildeten Elektrode bewirkt. Alternativ kann auch eine Suspension aus Keramik- und Glaspartikeln abgeschieden werden.

Die EP-A-0826642 offenbart ein Verfahren, bei dem zunächst aus einem Schlicker eine Keramikfolie hergestellt und die so erhaltene Keramikfolie hydrostatisch auf einen Grundkörper aufgepresst wird. Beim Herstellungsverfahren des dentalen Formteils wird die Keramikfolie großen mechanischen Belastungen ausgesetzt.

### Aufgabe und Lösung

Aufgabe der Erfindung ist es, ein dentales Formteil bzw. ein Verfahren zu dessen Herstellung zu schaffen, das gegenüber dem Stand der Technik verbesserte Eigenschaften aufweist. Zudem soll die Paßgenauigkeit bei der Weiterverarbeitung des dentalen Formteils verbessert werden.

Diese Aufgabe wird durch das Verfahren mit den Merkmalen des Anspruches 1, durch die Verwendung gemäss Anspruch 15 sowie durch die dentalen Formteile mit den Merkmalen der Ansprüche 16 und 19 gelöst. Weiterbildungen der Erfindung sind in den abhängigen Ansprüchen dargestellt.

Das Einbringen der keramischen Fasern bzw. Whisker in das Gefüge des dentalen Formteils bewirkt eine Verbesserung der mechanischen Eigenschaften, besonders hinsichtlich der Rißzähigkeit. Diese Fasern verbessern vor allem die elastische Steifigkeit des keramischen Werkstoffes. Zugleich läßt sich die Bruchfestigkeit durch Einbau von Fasern in eine Matrix erhöhen. Ein weiterer Vorteil einer derartigen Faserverbundkeramik liegt darin, daß ein etwaiger von der Oberfläche her eingeleiteter Riß nicht oder nur erschwert fortschreiten kann und daher nicht zu einem totalen Bruch führen muß.

Die nanokristallinen Partikel im Gefüge des dentalen Formteils bewirken eine hohe Sinteraktivität bei vergleichsweise niedrigeren Sintertemperaturen, sowie ebenfalls eine signifikante Verbesserung der mechanischen Eigenschaften. Bei kombinierter Anwendung von Keramikfasern und Nanopartikeln können besonders gute Ergebnisse erzielt werden.

Weiter umfaßt die Erfindung ein Verfahren zur Herstellung der beschriebenen dentalen Formteile durch elektophoretische Abscheidung von in einem flüssigen Dispersionsmittel dispergierten Keramikpartikeln. Dabei werden die Keramikpartikel aus der Dispersion an einer der Form eines dentalen Grundkörpers entsprechenden Elektrode abgeschieden. Der erhaltene Grünling wird zum Formteil gesintert.

Das erfindungsgemäße Verfahren zeichnet sich dadurch aus, daß es sich bei den Keramikpartikeln mindestens teilweise um keramische Fasern und/oder nanokristalline Partikel handelt.

Es eignet sich zur Herstellung von allen denkbaren keramischen Form- oder Gerüstteilen, wie Kronen, Brücken oder dergleichen. Eine keramische Krone beispielsweise besteht aus einem Unterbau und einer der Zahnform nachempfundenen sogenannten Verblendung aus Dentalkeramik. Der Unterbau wird beim Einsetzen des Zahnersatzes direkt auf dem nach der zahnärztlichen Präparation verbleibenden Restzahn befestigt und wird oft als Käppchen bezeichnet. Mit dem erfindungsgemäßen Verfahren ist es möglich, solche Käppchen herzustellen, die eine hohe Paßgenauigkeit beim Aufsetzen auf den verbleibenden Restzahn aufweisen.

Bei der elektrophoretischen Abscheidung ist es notwendig, zunächst einen keramischen Schlicker herzustellen. Unter keramischen Schlicker versteht man Suspensionen dispergierter keramischer Pulver in geeigneten flüssigen Dispersionsmitteln. Als Dispersionsmittel werden vorzugsweise polare Dispersionsmittel eingesetzt. Bevorzugtes Dispersionsmittel hierbei ist Wasser. Aber auch Alkohole, bevorzugt niedere Alkohole, sind als Dispersionsmittel geeignet.

Der keramischer Schlicker wird an einer der Form eines dentalen Grundkörpers entsprechenden Elektrode elektrophoretisch abgeschieden. Als dentaler Grundkörper kann ein Duplikat eines präparierten Zahnstumpfes eingesetzt werden. In den meisten Fällen wird bei der Elektrophorese eine anodische Abscheidung der dispergierten Feststoffpartikel beobachtet. Die Keramikpartikeln werden also negativ aufgeladen und wandern zum Pluspol, also zur vom dentalen Grundkörper gebildeten Anode. Es ist jedoch auch eine kathodische Abscheidung der Feststoffpartikel möglich.

Der Anteil der keramischen Fasern an der Gesamtmenge der Keramikpartikel ist vorzugsweise relativ gering. Er beträgt beispielsweise ca. 0,5 Gew. % bis 5 Gew. %, insbesondere 1 Gew. % bis 2 Gew. %. Der keramische Schlicker besteht also vorzugsweise hauptsächlich aus keramischen Pulverpartikeln, im folgenden Primärpartikel genannt, und einem geringen Anteil an keramischen Fasern. Die Primärpartikel besitzen einen mittleren Durchmesser, der im Bereich von 0,4 µm bis 0,8 µm, vorzugsweise 0,6 µm bis 0,7 µm liegt. Der mittlere Durchmesser der keramischen Fasern kann deutlich größer sein. Vorzugsweise liegt er im Bereich von 1 µm bis 20 µm, insbesondere 5 µm bis 10 µm. Die Länge der keramischen Fasern kann im Bereich von 0,1 mm bis 50 mm, insbesondere 5 mm bis 10 mm liegen. Besonders bevorzugt handelt es sich bei den keramischen Fasern, wie bei den Primärpartikeln, um solche aus der Gruppe der Oxidkeramiken. Die Primärpartikel bzw. Fasern können beispielsweise aus Aluminiumoxid und/oder Zirkoniumoxid bestehen. Das Zirkoniumoxid kann ggf. mit Yttriumoxid, Ceriumoxid o. dgl. stabilisiert sein.

Alternativ zu den keramischen Fasern sind keramische Schlicker mit nanokristallinen Partikeln einsetzbar. Es ist jedoch auch möglich keramische Fasern und nanokristalline Partikel einzusetzen, die gemeinsam abgeschieden werden.

Der Anteil der nanokristallinen Partikel an der Gesamtmenge der Keramikpartikel beträgt vorzugsweise 0,5 Gew. % bis 10 Gew. %, insbesondere 1 Gew. % bis 2 Gew. %. Die nanokristallinen Partikel sind im Vergleich zu den keramischen Primärpartikeln sehr viel kleiner. Der mittlere Durchmesser der nanokristallinen Partikel kann im Bereich von 1 nm bis 200 nm, insbesondere 5 nm bis 20 nm liegen. Wie die keramischen Primärpartikel, handelt es sich bei den nanokristallinen Partikeln vorzugsweise um solche aus der Gruppe der Oxidkeramiken. Die nanokristallinen Partikel können beispielsweise aus Aluminiumoxid und/oder Zirkoniumoxid sein.

Bei der Herstellung des keramischen Schlickers aus keramischen Primärpartikeln und keramischen Fasern und/oder nanokristallinen Partikeln können verschiedene Hilfsstoffe zugegeben werden. Zudem können der keramische Schlicker oder einzelne Bestandteile des Schlickers einer Vorbehandlung unterzogen werden. Der bevorzugte pH-Wert für die elektrophoretische Abscheidung liegt im Bereich von pH 5 bis 9. Gegebenenfalls kann der pH-Wert durch Zugabe einer Säure oder einer Base, beispielsweise Zitronensäure oder Natriumpyrophosphat, eingestellt werden. Um das Dispergieren der Keramikpartikel im Lösungsmittel zu verbessern, kann ein Dispergierhilfsmittel zugegeben werden. Als Dispergierhilfsmittel kann beispielsweise Natriumpyrophosphat dienen. Als keramische Primärpartikel kann eine Mischung aus Partikeln verschiedener Oxidkeramiken eingesetzt werden, die im festen Zustand vorgemischt werden. Beispielsweise können die Primärpartikel aus einer Feststoffmischung aus Aluminiumoxidpartikeln und Zirkoniumoxidpartikeln im Verhältnis 4:1 hergestellt werden. Die keramischen Primärpartikeln können unter Rühren in das Dispersionsmittel gegeben werden, und um das Dispergieren weiter zu verbessern, mit Ultraschall behandelt werden. Danach werden die keramischen Fasern und/oder die nanokristallinen Partikel beigemischt. Es ist aber auch denkbar, die keramischen Primärpartikeln und die keramischen Fasern und/oder nanokristallinen Partikel im festen Zustand vorzumischen und dann gemeinsam in das Dispersionsmittel zu geben. Um die Dispergierung weiter zu verbessern, insbesondere um die Fasern zu zerkleinern und vollständig zu dispergieren, kann der keramische Schlicker mit einem Dispergator behandelt werden. Vorzugsweise wird dem keramischen Schlicker ein organisches Additiv, beispielsweise ein mehrwertiger Alkohol, insbesondere Polyvinylalkohol, zugegeben, um beim Abscheiden eine bessere Agglomeration der Partikel zu erreichen. Um das Trocknen des abgeschiedenen keramischen Formteils zu erleichtern, kann dem keramischen Schlicker ein Trocknungshilfsmittel zugegeben werden. Das Trocknungsmittel kann ein Amid sein, beispielsweise Formamid.

Nach der Herstellung des Schlickers erfolgt dessen elektrophoretische Abscheidung an einer der Form eines dentalen Grundkörpers entsprechenden Elektrode. Bei der Elektrophorese wandern die dispergierten Keramikpartikel unter dem Einfluß eines elektrischen Feldes in eine bestimmte Richtung, die von ihrer Ladung bzw.

Oberflächenladung abhängt. Der keramische Schlicker wird also an der Elektrode, vorzugsweise anodisch abgeschieden. Grundsätzlich gibt es zwei Arten, die elektrophoretische Abscheidung durchzuführen. Sie kann bei konstanter Stromstärke und daraus resultierend zunehmender Spannung durchgeführt werden. Es ist aber auch möglich, bei konstanter Spannung und daraus resultierend abnehmender Stromstärke zu arbeiten. Bei konstanter Stromstärke wird diese aus einem Bereich von 1 mA bis 100 mA, vorzugsweise 10 mA bis 50 mA gewählt. Die Anfangsspannung liegt dabei im Bereich von 0,5 V bis 30 V, vorzugsweise 1 V bis 5 V. Bei konstanter Spannung wird diese aus einem Bereich von 1 V bis 100 V, vorzugsweise 2 V bis 10 V gewählt. Dabei beträgt die Anfangsstromstärke ca. 1 mA bis 100 mA, vorzugsweise 10 mA bis 50 mA.

Nach der elektrophoretischen Abscheidung kann das abgeschiedene Formteil nachbehandelt werden. Das abgeschiedene Formteil, das vor dem Brennen auch Keramikgrünling genannt wird, wird vorzugsweise zunächst getrocknet, beispielsweise mittels einer Mikrowelle. Die Keramikgrünlinge haben eine Gründichte von etwa 30 % bis 70 %, typischerweise etwa 50 % der theoretischen Dichte der Keramik.

Der wichtigste Nachbehandlungsschritt ist der Sintervorgang, bei dem die Dichte des dentalen Formteils auf über 90 % der theoretischen Dichte der Keramik erhöht werden kann. Je nach Zusammensetzung und Gehalt der dispergierten Pulver in den zur Abformung verwendeten Schlicker können die Grünlinge bei Temperaturen zwischen 700° und 1600°, insbesondere 1000° und 1400° gebrannt werden. Die Dauer des Sintervorganges kann mehrere Stunden, beispielsweise 2 bis 8, typischerweise ca. 5 Stunden lang dauern. Die Aufheizung auf die Sintertemperatur erfolgt vorzugsweise langsam, beispielsweise in einem Schritt von 1° bis 20° pro Minute, insbesondere etwa 5° bis 10° pro Minute.

Nach dem Sintern kann das gebrannte dentale Formteil mit Glaspartikeln infiltriert werden, um die beim Sintern entstandenen Poren im Gefüge zu verschließen.

Der Anteil der keramischen Fasern im Gefüge des dentalen Formteils liegt bei. 0,5 Gew. % bis 5 Gew. %, insbesondere 1 Gew. % bis 2 Gew. %. Der Anteil der nanokristallinen Partikel im Gefüge kann 0,5 Gew. % bis 10 Gew. %, insbesondere 1 Gew. % bis 2 Gew. % betragen.

Der mittlere Durchmesser der keramischen Fasern im Gefüge liegt vorzugsweise im Bereich von 1 µm bis 20 µm bzw. 5 µm bis 10 µm. Die Länge der keramischen Fasern im Gefüge kann im Bereich von 0,1 mm bis 5 mm, insbesondere 1 mm bis 2 mm liegen. Der mittlere Durchmesser der nanokristallinen Partikel liegt im Bereich von 1 nm bis 200 nm, insbesondere 5 nm bis 100 nm.

Wie bereits erwähnt, kann das dentale Formteil, dessen Gefüge keramische Primärpartikel und keramische Fasern bzw. keramische Primärpartikel und nanokristalline Partikel enthält, als kappenartiger Hohlformkörper ausgebildet sein. Er kann beispielsweise als Unterbau bzw. Käppchen einer keramischen Krone dienen. Die Wandstärke des dentalen Formteils kann 0,1 mm bis 1 mm, vorzugsweise 0,3 mm bis 0,7 mm betragen.

Die beschriebenen Merkmale und weitere Merkmale der Erfindung ergeben sich aus der nachfolgenden Beschreibung eines Beispiels in Verbindung mit den Unteransprüchen. Hierbei können die einzelnen Merkmale jeweils für sich oder in Kombination miteinander verwirklicht sein.

### Beispiel

Das erfindungsgemäße Verfahren zur Herstellung von keramischen Formteilen sowie das dentale Formteil soll beispielhaft anhand der Herstellung einer keramischen Krone erläutert werden.

### Herstellung des dentalen Grundkörpers (Elektrode)

Zunächst wird der dentale Grundkörper hergestellt, der bei der elektrophoretischen Abscheidung als Elektrode dient. Der dentale Grundkörper ist im beschriebenen Beispiel ein Duplikatstumpf eines Zahnes, der elektrisch z. B. mit Leitsilberlack kontaktiert ist und als Elektrode in einem Stromkreis geschaltet ist. Zur Herstellung des Duplikatstumpfes wird zunächst ein Negativabdruck des Zahnes, beispielsweise mit einer Silikonmasse, angefertigt. Mit Hilfe dieses Negativabdruckes kann der Duplikatstumpf angefertigt werden, beispielsweise gegossen werden. Als Stumpfmaterial werden dabei bei den Brenntemperaturen stabile Materialien, bevorzugt in der Zahntechnik gebräuchliche sogenannte feuerfeste Einbettmassen gewählt, wie sie beispielsweise in der dentalen Gußtechnik verwendet werden, so z. B. gipsgebundene Einbettmassen oder Löteinbettmassen.

### Schlickerherstellung mit keramischen Primärpartikeln und keramischen Fasern:

Zunächst werden zu 250 ml Wasser je 0,2 g Natriumpyrophospat und Citronensäure zugegeben und gerührt. Dann werden bei leichter Erwärmung 100 g eines aus Oxiden gemischten Keramikpulvers (Al₂O₃ : ZrO₂ = 4 : 1) portionsweise zugegeben und intensiv gerührt. Danach werden, nach Ultraschallbehandlung der Dispersion 4 g = 4 Gew. % ZrO₂ - bzw. Al₂O₃-Fasern zu dem Schlicker gegeben und mehrere Stunden kräftig gerührt und anschließend etwa 30 min. mit einem Heidolph Dispergator "DIAX 900" behandelt, wobei gleichzeitig intensiv gerührt wird, bis die Fasern vollständig dispergiert sind. Anschließend werden der Dispersion 12 ml der 5%igen PVA-Lösung, MG 72.000 langsam eingerührt, nach einiger Zeit noch 6 ml Formamid als DCCA (= Trocknungshilfsmittel) zugegeben und dann über Nacht intensiv gerührt. Verwendete Chemikalien: Aluminiumoxidpulver CT 3000 SG, Fa. ALCOA; Zirkonoxidpulver SC 15, MEL CHEMICALS; Citronensäure-Monohydrat 99,5 %, Fa. MERCK; Natriumpyrophosphat-Dekahydrat, Fa. RIEDEL DE HAEN; Polyvinylalkohol, Molekulargewicht 72.000, Fa. FLUKA; Formamid 99,5 %, Fa. ALDRICH; Almax-Fasern (Al₂O₃-Fasern) der Fa. MITSUI; ZrO₂-Fasern, Fa. SINDLHAUSER MATERIALS.

### Schlickerherstellung mit keramischen Primärpartikeln und nanokristallinen Partikeln

Zunächst werden zu 250 ml Wasser je 0,2 g Natriumpyrophospat und Citronensäure zugegeben und gerührt. Dann werden bei leichter Erwärmung 100 g eines aus Oxiden gemischten Keramikpulvers (Al₂O₃ : ZrO₂ = 4 : 1) portionsweise zugegeben und intensiv gerührt. Danach werden, nach Ultraschallbehandlung der Dispersion 4 g Aluminiumoxid C (entspricht 4 Gew. %) zu dem Schlicker gegeben und mehrere Stunden kräftig gerührt und anschließend etwa 30 min. mit einem Heidolph Dispergator "DIAX 900" behandelt, wobei gleichzeitig intensiv gerührt wird. Anschließend werden der Dispersion 12 ml der 5%igen PVA-Lösung, MG 72.000 langsam eingerührt, nach einiger Zeit noch 6 ml Formamid als DCCA (= Trocknungshilfsmittel) zugegeben und dann über Nacht intensiv gerührt.

Verwendete Chemikalien: Aluminiumoxidpulver CT 3000 SG, Fa. ALCOA; Zirkonoxidpulver SC 15, MEL CHEMICALS; Citronensäure-Monohydrat 99,5 %, Fa. MERCK; Natriumpyrophosphat-Dekahydrat, Fa. RIEDEL DE HAEN; Polyvinylalkohol, Molekulargewicht 72.000, Fa. FLUKA; Formamid 99,5 %, Fa. ALDRICH; Aluminiumoxid C, DEGUSSA AG.

### Elektrophoretische Abscheidung

In ein 100 ml-Laborbecherglas werden etwa 70 ml der oben beschriebenen Schlicker eingefüllt und etwa 5 min. einer Ultraschallbehandlung unterzogen. Dann wird, unter langsamen Rühren (ca. 100 U/min.) mit Hilfe einer Gleichstromquelle, mit einem Platinblech als Kathode und einem mit Leitsilberlack kontaktierten Modell-Zahnstumpf aus feuerfester Stumpfmasse (L 36, Fa. HINRICHS) als Anode bei typischen Spannungen von 1 - 5 V und Strömen von 20 - 100 mA in einer Prozeßdauer von typischerweise etwa 10 - 15 Minuten die keramischen Bestandteile des Schlickers in Form einer glatten, optisch dichten, etwa 0,4 mm bis 0,5 mm dicken Schicht (Grünkörper) abgeschieden.

### Sintervorgang

Die Modellstümpfe mit elektrophoretisch abgeschiedener Keramikschicht werden getrocknet und anschließend in einem Laborofen (HAT 1660, Fa. NABERTHERM) einem Sinterbrand unterzogen. Nach Abscheidung aus rein keramischen Schlickern, also keramischen Primärpartikeln und keramischen Fasern und/oder keramischen nanokristallinen Partikeln wird vorzugsweise mit folgenden Parametern gearbeitet:
- Aufheizrate bis 120°C: 2°C/min
- Aufheizrate bis 1150°C: 7,5°C/min
- Haltezeit 5 h bei 1150°C, dann im Ofen auskühlen

## Patentansprüche

1. Verfahren zur Herstellung von keramischen Formteilen für dentale Anwendungen, bei dem in einem flüssigen Disperionsmittel dispergierte Keramikpartikel aus der Dispersion an einer der Form eines dentalen Grundkörpers entsprechenden Elektrode elektrophoretisch abgeschieden werden und der erhaltene Grünling gesintert wird, **dadurch gekennzeichnet, daß** es sich bei den Keramikpartikeln mindestens teilweise um keramische Fasern und/ oder nanokristalline Partikel handelt.

2. Verfahren nach Anspruch 1, **dadurch gekennzeichnet, daß** der Anteil der keramischen Fasern an der Gesamtmenge der Keramikpartikel im Bereich von 0,5 Gew.-% bis 5 Gew.-%, insbesondere von 1 Gew.-% bis 2 Gew.-%, beträgt.

3. Verfahren nach Anspruch 1 oder 2, **dadurch gekennzeichnet, daß** der mittlere Durchmesser der keramischen Fasern im Bereich von 1 µm bis 20 µm, insbesondere im Bereich von 5 µm bis 10 µm, liegt.

4. Verfahren nach einem der vorhergehenden Ansprüche, **dadurch gekennzeichnet, daß** die Länge der keramischen Fasern im Bereich von 0,1 mm bis 50 mm, insbesondere 5 mm bis 10 mm, liegt.

5. Verfahren nach einem der vorhergehenden Ansprüche, **dadurch gekennzeichnet, daß** der Anteil der nanokristallinen Partikel an der Gesamtmenge der Keramikpartikel im Bereich von 0,5 Gew.-% bis 10 Gew.-%, insbesondere von 1 Gew.-% bis 2 Gew.-%, beträgt.

6. Verfahren nach einem der vorhergehenden Ansprüche, **dadurch gekennzeichnet, daß** der mittlere Durchmesser der nanokristallinen Partikel im Bereich von 1 nm bis 200 nm, insbesondere 5 nm bis 20 nm, liegt.

7. Verfahren nach einem der vorhergehenden Ansprüche, **dadurch gekennzeichnet, daß** es sich bei den keramischen Fasern bzw. nanokristallinen Partikeln um solche aus der Gruppe der Oxidkeramiken, insbesondere um keramische Aluminiumoxid- und/oder Zirkoniumoxidfasern bzw. nanokristalline Aluminiumoxid- und/oder Zirkoniumoxidpartikel handelt.

8. Verfahren nach einem der vorhergehenden Ansprüche, **dadurch gekennzeichnet, daß** es sich bei dem Dispersionsmittel um mindestens ein polares Dispersionsmittel, insbesondere um Wasser, handelt.

9. Verfahren nach einem der vorhergehenden Ansprüche, **dadurch gekennzeichnet, daß** die Dispersion mindestens ein organisches Additiv, vorzugsweise mindestens einen mehrwertigen Alkohol, insbesondere Polyvinylalkohol, enthält.

10. Verfahren nach einem der vorhergehenden Ansprüche, **dadurch gekennzeichnet, daß** die Dispersion mindestens ein Trocknungshilfsmittel, vorzugsweise mindestens ein Amid, insbesondere Formamid, enthält

11. Verfahren nach einem der vorhergehenden Ansprüche, **dadurch gekennzeichnet, daß** die elektrophoretische Abscheidung bei konstanter Stromstärke bzw. konstanter Spannung und daraus resultierend, zunehmender Spannung bzw. abnehmender Stromstärke durchgeführt wird.

12. Verfahren nach einem der vorhergehenden Ansprüche, **dadurch gekennzeichnet, daß** die Stromstärke bzw. Spannung aus einem Bereich von 1 mA bis 100 mA bzw. 1 V bis 100 V, vorzugsweise von 10 mA bis 50 mA bzw. 2 V bis 10 V, gewählt wird.

13. Verfahren nach einem der vorhergehenden Ansprüche, **dadurch gekennzeichnet, daß** die Anfangsspannung bzw. Anfangsstromstärke im Bereich von 0,5 V bis 30 V bzw. 1 mA bis 100 mA, vorzugsweise von 1 V bis 5 V bzw. 10 mA bis 50 mA, gewählt wird.

14. Verfahren nach einem der vorhergehenden Ansprüche, **dadurch gekennzeichnet, daß** die Sintertemperatur zwischen 700 °C und 1600 °C, insbesondere zwischen 1000 °C und 1400 °C, beträgt.

15. Verwendung von keramischen Fasern und/oder nanokristallinen Keramikpartikeln zur Herstellung vollkeramischer dentaler Formkörper durch elektrophoretische Abscheidung.

16. Dentales Formteil herstellbar aus Keramikpartikeln und Brennen des daraus erhaltenen Grünlings, wobei es im Gefüge keramische Fasern enthält, **dadurch gekennzeichnet, daß** der Anteil der keramischen Fasern im Gefüge 0,5 Gew-% bis 5 Gew.-%, insbesondere 1 Gew.-% bis 2 Gew.-%, beträgt.

17. Dentales Formteil nach Anspruch 16, **dadurch gekenzeichnet, daß** der mittlere Durchmesser der keramischen Fasern im Gefüge im Bereich von 1 µm bis 20 µm, vorzugsweise 5 µm bis 10 µm, liegt.

18. Dentales Formteil nach Anspruch 16 oder 17, **dadurch gekennzeichnet, daß** die Länge der keramischen Fasem im Gefüge im Bereich von 0,1 mm bis 5 mm, insbesondere 1 mm bis 2 mm, liegt.

19. Dentales Formteil herstellbar aus Keramikpartikeln und Brennen des daraus erhaltenen Grünlings, wobei es im Gefüge nanokristalline Partikel enthält, **dadurch gekennzeichnet, daß** der mittlere Durchmesser der nanokristallinen Partikel im Bereich von 1 nm bis 200 nm, insbesondere 5 nm bis 20 nm, liegt.

20. Dentales Formteil nach Anspruch 19, **dadurch gekennzeichnet, daß** der Anteil der nanokristallinen Partikel im Gefüge 0,5 Gew.-% bis 10 Gew.-%, insbesondere 1 Gew.-% bis 2 Gew.-%, beträgt.

21. Dentales Formteil nach einem der Ansprüche 16 bis 20, **dadurch gekennzeichnet, daß** der Grünling durch elektrophoretische Abscheidung von Keramikpartikeln an einer der Form eines dentalen Grundkörpers entsprechenden Elektrode erhältlich ist.

22. Dentales Formteil nach einem der Ansprüche 16 bis 21, **dadurch gekennzeichnet, daß** es sich bei den keramischen Fasern bzw. nanokristallinen Partikeln um solche aus der Gruppe der Oxidkeramiken, insbesondere um Aluminiumoxid- und/oder Zirkoniumoxidfasern bzw. nanokristalline Aluminiumoxid- und/oder Zirkoniumoxidpartikel handelt.

23. Dentales Formteil nach einem der Ansprüche 16 bis 22, **dadurch gekennzeichnet, daß** es als, vorzugsweise kappenartiger, Hohlformkörper ausgebildet ist, der insbesondere eine Wandstärke von 0,1 mm bis 1 mm, vorzugsweise 0,3 mm bis 0,7 mm aufweist.

## Claims

1. A method for producing shaped ceramic parts for dental applications, in which ceramic particles dispersed in a liquid dispersion medium are electrophoretically deposited from the dispersion onto an electrode corresponding to the shape of a dental base body and the green body obtained is sintered, **characterized in that** the ceramic particles are at least partly ceramic fibres and/or nanocrystalline particles.

2. The method according to Claim 1, **characterized in that** the proportion of ceramic fibres based on the total amount of ceramic particles is in the range from 0.5% by weight to 5% by weight, in particular from 1% by weight to 2% by weight.

3. The method according to Claim 1 or 2,
**characterized in that** the average diameter of the ceramic fibres is in the range from 1 µm to 20 µm, in particular in the range from 5 µm to 10 µm.

4. The method according to any of the preceding claims, **characterized in that** the length of the ceramic fibres is in the range from 0.1 mm to 50 mm, in particular from 5 mm to 10 mm.

5. The method according to any of the preceding claims, **characterized in that** the proportion of nanocrystalline particles based on the total amount of ceramic particles is in the range from 0.5% by weight to 10% by weight, in particular from 1% by weight to 2% by weight.

6. The method according to any of the preceding claims, **characterized in that** the average diameter of the nanocrystalline particles is in the range from 1 nm to 200 nm, in particular from 5 nm to 20 nm.

7. The method according to any of the preceding claims, **characterized in that** the ceramic fibres or nanocrystalline particles are fibres or particles selected from the group of oxide ceramics, in particular ceramic aluminium oxide and/or zirconium oxide fibres or nanocrystalline aluminium oxide and/or zirconium oxide particles.

8. The method according to any of the preceding claims, **characterized in that** the dispersion medium is at least one polar dispersion medium, in particular water.

9. The method according to any of the preceding claims, **characterized in that** the dispersion contains at least one organic additive, preferably at least one polyhydric alcohol, in particular polyvinyl alcohol.

10. The method according to any of the preceding claims, **characterized in that** the dispersion contains at least one drying aid, preferably at least one amide, in particular formamide.

11. The method according to any of the preceding claims, **characterized in that** the electrophoretic deposition is carried out at a constant current or constant voltage and, resulting therefrom, increasing voltage or decreasing current.

12. The method according to any of the preceding claims, **characterized in that** the current or voltage is selected in the range from 1 mA to 100 mA and from 1 V to 100 V, preferably from 10 mA to 50 mA and from 2 V to 10 V, respectively.

13. The method according to any of the preceding claims, **characterized in that** the initial voltage or initial current is selected in the range from 0.5 V to 30 V and from 1 mA to 100 mA, preferably from 1 V to 5 V and from 10 mA to 50 mA, respectively.

14. The method according to any of the preceding claims, **characterized in that** the sintering temperature is in the range from 700°C to 1600°C, in particular from 1000°C to 1400°C.

15. Use of ceramic fibres and/or nanocrystalline ceramic particles for producing all-ceramic dental moulded bodies by electrophoretic deposition.

16. Dental moulded part which can be produced from ceramic particles and firing of the green body obtained therefrom and contains ceramic fibres in the microstructure, **characterized in that** the proportion of ceramic fibres in the microstructure is from 0.5% by weight to 5% by weight, in particular from 1% by weight to 2% by weight.

17. Dental moulded part according to Claim 16, **characterized in that** the average diameter of the ceramic fibres in the microstructure is in the range from 1 µm to 20 µm, preferably from 5 µm to 10 µm.

18. Dental moulded part according to Claim 16 or 17, **characterized in that** the length of the ceramic fibres in the microstructure is in the range from 0.1 mm to 5 mm, in particular from 1 mm to 2 mm.

19. Dental moulded part which can be produced from ceramic particles and firing of the green body obtained therefrom and contains nanocrystalline particles in the microstructure, **characterized in that** the average diameter of the nanocrystalline particles is in the range from 1 nm to 200 nm, in particular from 5 nm to 20 nm.

20. Dental moulded part according to Claim 19, **characterized in that** the proportion of nanocrystalline particles in the microstructure is from 0.5% by weight to 10% by weight, in particular from 1% by weight to 2% by weight.

21. Dental moulded part according to any of Claims 16 to 20, **characterized in that** the green body can be obtained by electrophoretic deposition of ceramic particles on an electrode corresponding to the shape of a dental base body.

22. Dental moulded part according to any of Claims 16 to 21, **characterized in that** the ceramic fibres or nanocrystalline particles are fibres or particles selected from the group of oxide ceramics, in particular aluminium oxide and/or zirconium oxide fibres or nanocrystalline aluminium oxide and/or zirconium oxide particles.

23. Dental moulded part according to any of Claims 16 to 22, **characterized in that** it is configured as a preferably cap-like, hollow shaped body which has, in particular, a wall thickness of from 0.1 mm to 1 mm, preferably from 0.3 mm to 0.7 mm.

## Revendications

1. Procédé de fabrication de pièces moulées en céramique pour applications dentaires, dans lequel des particules de céramique dispersées dans un milieu liquide de dispersion sont déposées par électrophorèse depuis la dispersion sur une électrode dont la forme correspond à celle d'un corps dentaire de base, l'ébauche ainsi obtenue étant frittée, **caractérisé en ce que** les particules de céramique sont au moins en partie des fibres céramiques et/ou des particules nanocristallines.

2. Procédé selon la revendication 1, **caractérisé en ce que** la proportion des fibres céramiques par rapport à la quantité totale de particules en céramique est comprise dans la plage de 0,5 % en poids à 5 % en poids et en particulier de 1 % en poids à 2 % en poids.

3. Procédé selon les revendications 1 ou 2, **caractérisé en ce que** le diamètre moyen des fibres céramiques est compris dans la plage de 1 µm à 20 µm et en particulier dans la plage de 5 µm à 10 µm.

4. Procédé selon l'une des revendications précédentes, **caractérisé en ce que** la longueur des fibres céramiques est comprise dans la plage de 0,1 mm à 50 mm et en particulier de 5 mm à 10 mm.

5. Procédé selon l'une des revendications précédentes, **caractérisé en ce que** la proportion des particules nanocristallines par rapport à la quantité totale des particules céramiques est comprise dans la plage de 0,5 % en poids à 10 % en poids et en particulier de 1 % en poids à 2 % en poids.

6. Procédé selon l'une des revendications précédentes, **caractérisé en ce que** le diamètre moyen des particules nanocristallines est compris dans la plage de 1 nm à 200 nm et en particulier de 5 nm à 20 nm.

7. Procédé selon l'une des revendications précédentes, **caractérisé en ce que** les fibres céramiques ou les particules cristallines sont sélectionnées dans l'ensemble constitué des céramiques d'oxyde et en particulier des fibres céramiques d'oxyde d'aluminium et/ou d'oxyde de zirconium et des particules nanocristallines d'oxyde d'aluminium et/ou d'oxyde de zirconium.

8. Procédé selon l'une des revendications précédentes, **caractérisé en ce que** le fluide de dispersion est au moins un fluide de dispersion polaire et en particulier l'eau.

9. Procédé selon l'une des revendications précédentes, **caractérisé en ce que** la dispersion contient au moins un additif organique et de préférence au moins un alcool polyfonctionnel et en particulier un alcool polyvinylique.

10. Procédé selon l'une des revendications précédentes, **caractérisé en ce que** la dispersion contient au moins un adjuvant de séchage et de préférence au moins un amide et en particulier le formamide.

11. Procédé selon l'une des revendications précédentes, **caractérisé en ce que** la séparation par électrophorèse est réalisée à intensité de courant constante ou à tension constante, avec par conséquent une tension croissante ou une intensité de courant décroissante.

12. Procédé selon l'une des revendications précédentes, **caractérisé en ce que** l'intensité du courant et la tension sont sélectionnées respectivement dans les plages de 1 mA à 100 mA et de 1 V à 100 V et de préférence dans les plages de 10 mA à 50 mA et de 2 V à 10 V.

13. Procédé selon l'une des revendications précédentes, **caractérisé en ce que** la tension initiale ou l'intensité initiale du courant sont sélectionnées respectivement dans les plages de 0,5 V à 30 V et de 1 mA à 100 mA et de préférence dans les plages de 1 V à 5 V et de 10 mA à 50 mA.

14. Procédé selon l'une des revendications précédentes, **caractérisé en ce que** la température de frittage est comprise entre 700 °C et 1 600 °C et en particulier entre 1 000°C et 1 400°C.

15. Utilisation de fibres céramiques et/ou de particules nanocristallines de céramique pour la fabrication de corps dentaires moulés en céramique pleins par dépôt par électrophorèse.

16. Pièce dentaire moulée fabriquée à partir de particules de céramique et cuisson de l'ébauche ainsi obtenue, et qui contient des fibres céramiques dans son réseau, **caractérisée en ce que** la proportion des fibres céramiques dans le réseau est comprise entre 0,5 % en poids et 5 % en poids et en particulier entre 1 % en poids et 2 % en poids.

17. Pièce dentaire moulée selon la revendication 16, **caractérisée en ce que** le diamètre moyen des fibres céramiques dans le réseau est compris dans la plage de 1 µm à 20 µm et de préférence de 5 µm à 10 µm.

18. Pièce dentaire moulée selon les revendications 16 ou 17, **caractérisée en ce que** la longueur des fibres céramiques dans le réseau est comprise dans la plage de 0,1 mm à 5 mm et en particulier de 1 mm à 2 mm.

19. Pièce dentaire moulée fabriquée à partir de particules de céramique et cuisson de l'ébauche ainsi obtenue, et contenant des particules nanocristallines dans son réseau, **caractérisée en ce que** le diamètre moyen des particules nanocristallines est compris dans la plage de 1 nm à 200 nm et en particulier de 5 nm à 20 nm.

20. Pièce dentaire moulée selon la revendication 19, **caractérisée en ce que** la proportion des particules nanocristallines dans le réseau est comprise entre 0,5 % en poids et 10 % en poids et en particulier entre 1 % en poids et 2 % en poids.

21. Pièce dentaire moulée selon l'une des revendications 16 à 20, **caractérisée en ce que** l'ébauche est obtenue par dépôt par électrophorèse de particules de céramique sur une électrode dont la forme correspond à celle d'un corps dentaire de base.

22. Pièce dentaire moulée l'une des revendications 16 à 21, **caractérisée en ce que** les fibres céramiques et les particules nanocristallines sont sélectionnées dans l'ensemble constitué des céramiques d'oxyde et en particulier des fibres d'oxyde d'aluminium et/ou d'oxyde de zirconium et des particules nanocristallines d'oxyde d'aluminium et/ou d'oxyde de zirconium.

23. Pièce dentaire moulée l'une des revendications 16 à 22, **caractérisée en ce qu'**elle est configurée comme corps moulé creux, en particulier en forme de coiffe, dont la paroi a en particulier une épaisseur de 0,1 mm à 1 mm et de préférence de 0,3 mm à 0,7 mm.
